# EUROPEAN PATENT SPECIFICATION

(11) **EP 4 453 894 B1**
(45) Date of publication and mention of the grant of the patent: **01.04.2026**
(21) Application number: 22839373.2
(22) Date of filing: 23.12.2022
(51) Int. Cl.: G06V 10/25, G06V 10/20, G06V 10/82, G16H 50/20, G16H 30/40

(54) **BLAST CELL CLASSIFICATION**
BLASTENKLASSIFIZIERUNG
CLASSIFICATION DES CELLULES BLASTIQUES

(30) Priority: 24.12.2021 EP 21217712
(43) Date of publication of application: 30.10.2024
(73) Proprietor: F. Hoffmann-La Roche AG, 4070 Basel (CH); Roche Diagnostics GmbH, 68305 Mannheim (DE)
(72) Inventor: BRUENGGEL, Nils, 6343 Rotkreuz (CH); CONWAY, Patrick, 6343 Rotkreuz (CH); DAVIDSON, Simon John, 6343 Rotkreuz (CH); DEJEAN, Emilie, 6343 Rotkreuz (CH); GILDENBLAT, Jacob, 2622, Ra'anana (IL); SAGIV, Chen, 2622, Ra'anana (IL); VALLOTTON, Pascal, 6343 Rotkreuz (CH)
(74) Representative: Mewburn Ellis LLP
(86) International application number: PCT/EP2022/087779
(87) International publication number: WO 2023/118586

(56) References cited:
- BOLDÚ LAURA ET AL: "A deep learning model (ALNet) for the diagnosis of acute leukaemia lineage using peripheral blood cell images", COMPUTER METHODS AND PROGRAMS IN BIOMEDICINE., vol. 202, 12 February 2021 (2021-02-12), NL, pages 105999, XP055925563, ISSN: 0169-2607, DOI: 10.1016/j.cmpb.2021.105999
- ZHOU MIN ET AL: "Development and Evaluation of a Leukemia Diagnosis System Using Deep Learning in Real Clinical Scenarios", FRONTIERS IN PEDIATRICS, vol. 9, 24 June 2021 (2021-06-24), CH, pages 693676.1, XP055925524, ISSN: 2296-2360, DOI: 10.3389/fped.2021.693676
- CHEN DECHANG ET AL: "An asymptotic analysis of some expert fusion methods", PATTERN RECOGNITION LETTERS., vol. 22, no. 8, June 2001 (2001-06-01), NL, pages 901 - 904, XP093028977, ISSN: 0167-8655, DOI: 10.1016/S0167-8655(01)00031-9
- ROLLINS-RAVAL MARIAN A. ET AL: "Experience with CellaVision DM96 for peripheral blood differentials in a large multi-center academic hospital system", JOURNAL OF PATHOLOGY INFORMATICS, vol. 3, no. 1, 25 August 2012 (2012-08-25), IN, pages 29, XP093028936, ISSN: 2153-3539, DOI: 10.4103/2153-3539.100154
- KAREN SIMONYAN ET AL: "Very Deep Convolutional Networks for Large-Scale Image Recognition", 4 September 2014 (2014-09-04), XP055270857, Retrieved from the Internet <URL:http://arxiv.org/pdf/1409.1556v6.pdf> [retrieved on 20160506]
- NIKITAEV V G ET AL: "The blood smear image processing for the acute leukemia diagnostics", INTERNATIONAL JOURNAL OF BIOLOGY AND BIOMEDICAL ENGINEERING, vol. 10, 2016, pages 109 - 114, XP055925451, ISSN: 1998-4510
- KAIMING HE ET AL: "Deep Residual Learning for Image Recognition", 2016 IEEE CONFERENCE ON COMPUTER VISION AND PATTERN RECOGNITION (CVPR), June 2016 (2016-06-01), pages 770 - 778, XP055536240, ISBN: 978-1-4673-8851-1, DOI: 10.1109/CVPR.2016.90

## Description

### TECHNICAL FIELD OF THE INVENTION

The present invention relates to computer-implemented methods of differentiating myeloid and lymphoid blast cells, and related methods for diagnosing acute myeloid leukaemia or acute lymphoid leukaemia based on the output of the differentiation. Computer-implemented methods for training a deep neural network are also provided, as well as a clinical decision support system.

### BACKGROUND TO THE INVENTION

Blast cells are precursors to mature blood cells which are found circulating in a person's bloodstream. Normally, blast cells are confined to a person's bone marrow. However, when a patient suffers from leukaemia, abnormal blast cells proliferate uncontrollably in the bone marrow to such an extent that production of other cells, important for survival, is prevented. Furthermore, the uncontrollable proliferation also causes the abnormal blast cells to leak into a person's bloodstream. Accordingly, leukaemia may be diagnosed by detection of these abnormal blast cells within a patient's blood stream.

Acute leukaemia presents itself in forms including acute myeloid leukaemia (AML) and acute lymphoid leukaemia (ALL), each of which have several subtypes. In order to determine which type of leukaemia is present, it is necessary to classify the abnormal blast cells as either myeloid blast cells or lymphoid blast cells. Up to this point, this classification has been very challenging, because the cells are immature, and lack cell lineage differentiation.

Current methods for classifying whether the abnormal blast cells are myeloid blast cells or lymphoid blast cells rely on several stages of cluster of differentiation (CD) marker assessments. An example of a workflow which might be used is as follows. In a first step, a complete blood count (CBC) would be obtained, either as part of routine screening, or due to the expression of possible symptoms of leukaemia. If the results of the CBC show abnormal results (i.e. abnormal numbers of blast cells in the blood), then a blood smear may be taken, and examined by a haematologist. If the presence of abnormal blast cells in the blood is confirmed in the analysis of the blood smear, repeat samples may be taken for confirmation. Then, further analysis including CD marker assessment may be performed. The CD marker assessment is used to determine cell lineage (i.e. whether the blast cells are myeloid blast cells or lymphoid blast cells). After that determination has taken place, a CD marker panel for specific myeloid or lymphoid cell lines may be carried out, eventually leading to a diagnosis. It will be appreciated that this is a lengthy process which requires several stages of CD marker assessment in order to reach a diagnosis. In combination with the CD marker assessment, other techniques may be used including analysis of cerebrospinal fluid (CSF) or bone marrow samples using cytogenetics, fluorescence *in situ* hybridization (FISH) or polymerase chain reaction (PCR) techniques. These processes are equally time consuming, and in many cases, expensive.

The present invention aims to address this by providing a computer-implemented method for differentiating between myeloid blast cells and lymphoid blast cells.

The prior art includes the method, which is described in Laura Boldú et al: "A deep learning model (ALNet) for the diagnosis of acute leukaemia lineage using peripheral blood cell images", Computer Methods and Programs in Biomedicine, vol. 202, 12 February 2021, page 105999, ISSN: 0169-2607, DOI: 10.1016/j.cmpb.2021.105999. The method applies first and second deep neural network classifiers. The first deep neural network classifier is a VGG16 network, which recognises abnormal promyelocytes among other mononuclear blood cell images, such as lymphocytes, monocytes, reactive lymphocytes and blasts. The second deep neural network classifier is a VGG19 network, which distinguishes if blasts are myeloid or lymphoid lineage.

### SUMMARY OF THE INVENTION

At a high level, the present invention provides a computer-implemented method which uses a parametric model to differentiate between lymphoid blast cells and myeloid blast cells. Alternatively put, the computer-implemented method is a method of determining whether a blast cell in a digital image is from a myeloid lineage or a lymphoid lineage. More specifically, a first aspect of the present invention provides a computer-implemented method of differentiating between lymphoid blast cells and myeloid blast cells, the computer-implemented method comprising: receiving a digital image containing one or more blast cells; applying a parametric model classifier to one or more portions of the digital image each containing a respective blast cell, the parametric model classifier configured to generate an output indicative of whether each blast cell is a lymphoid blast cell or a myeloid blast cell.

By using a classifier at an initial stage, as required by computer-implemented methods according to the first aspect of the invention, it is possible to reduce or avoid the need for various stages of CD marker analysis (or other lengthy techniques) in order to arrive at a definitive diagnosis of AML or ALL.

Optional features of the first aspect of the invention are now set out. It should be noted that any or all of these features may be combined with each other, except when context dictates otherwise, or when it is explicitly stated that a certain feature is incompatible or otherwise cannot be combined with another feature.

The digital image may be received from an imaging apparatus. And, in addition to the computer-implemented steps, a method according to the first aspect of the invention may comprise capturing the digital image of the one or more blast cells using the imaging apparatus. The imaging apparatus may comprise a camera. The imaging apparatus may further comprise a microscope. Accordingly, the digital image may be a microscopy image such as a bright-field microscopy image. Alternatively, however, the digital image could be based on phase contrast imaging, differential interference contrast microscopy, or dark field microscopy.

The digital image of the one or more blast cells may be a digital image of a sample on a slide.

In addition to the computer-implemented steps, a method according to the first aspect of the invention may further comprise preparing a slide containing one or more blast cells.

Preferably, the slide is prepared using a method in which a drop of blood is allowed to dry in the presence of air, or in a moderate air flow, and using a technique which creates a monolayer of all of the cells in a volume transferred to a slide. Again preferably, the slide is prepared such that every single cell can be counted and differentiated by type. In order to ensure this, a proper diluent, dilution factor, means of allowing the drop to spread over a relatively large area by improving the hydrophilic nature of the glass slide and/or by mechanically spreading the liquid drop must be selected. Appropriate selection of parameters and methods gives rise to a very thin layer of liquid that rapidly dries in such a way that the presentation and preservation of the cells mimics that found in a conventional blood "smear". Details of methods by which advantageous slides may be prepared may be found in WO 2012/030313 A1. Additional disclosure regarding the preparation of the slides may be found in US 2009/0269799 A1, US 2010/284602 A1, US 2011/014645 A1, and US 2016/209320 A1.

As will be noted from the references above, it is preferred that the sample is stained. Accordingly, the digital image may be an image of a stained sample on a slide. Stains which may be used include: Romanowsky staining, Giemsa staining, Jenner staining, Wright staining, Field staining, May-Grünwald staining, and Leishman staining. It will be appreciated that other kinds of stains may also be used.

The computer-implemented method may further comprise, after receiving the digital image containing one or more blast cells: identifying the one or more blast cells in the digital image. In other words, before applying the classifier to each of the blast cells, the computer-implemented method may further comprise locating where the blast cells are in the image. Even in patients suffering from AML or ALL, the proportion of blast cells in the blood is still very low, relative to e.g. red blood cells. It is therefore beneficial to identify the blast cells within the digital image before applying the classifier, to ensure that the classifier acts only on the identified blast cells. Identifying the blast cells may comprise applying an image analysis algorithm to the digital image. The image analysis algorithm is preferably configured to identify bounding boxes around the one or more blast cells in the digital image. Specifically, the image analysis algorithm is preferably configured to identify a respective bounding box around each of the one or more blast cells in the digital image. Herein, identifying a bounding box should be understood to mean identifying an area, preferably a square or rectangular area which contains preferably a single blast cell. In some cases, the image analysis algorithm may be configured to generate a plurality of image files, each image file containing a digital image of a blast cell of the one or more blast cells. The boundary of the image in each image file of the plurality of image files may be the bounding box described above. In some cases, the plurality of files may be generated after the image analysis algorithm has been applied to the digital image. In the cases in which a plurality of image files are generated, the computer-implemented method may comprise applying the parametric model classifier to each of the generated image files, and configured to generate a respective output indicative of whether the respective blast cell in each image is a lymphoid blast cell or a myeloid blast cell. The output of the computer-implemented method may therefore comprise a plurality of outputs, each indicative of whether a respective blast cell of the one or more blast cells is a lymphoid blast cell or a myeloid blast cell.

We now consider in more detail the form of the output of the parametric model classifier. For each blast cell in the digital image, or for each image file, the output of the parametric model classifier may comprise a numerical value x indicative of whether the blast cell is a lymphoid blast cell or a myeloid blast cell. In some cases, the output (again, for each blast cell in the digital image, or for each image file) may comprise a first value and second value, the first value indicative of the likelihood that the blast cell is a lymphoid blast cell (or that the image file contains an image of a lymphoid blast cell), the second value indicative of the likelihood that the blast cell is a myeloid blast cell (or that the image file contains an image of a myeloid blast cell). Preferably, the first and the second value are probabilities, and preferably they sum to 1. The more extreme the values, the higher the confidence in the result. Put alternatively, the numerical value x may be in the range [0,1]; if the value x is equal to 1, the blast cell may be identified as a lymphoid blast cell with 100% confidence; and if the value x is equal to 0, the blast cell may be identified as a type of cell other than a lymphoid blast cell with 100% confidence. Or, the numerical value x may be in the range [0,1]; if the value x is equal to 1, the blast cell may be identified as a myeloid blast cell with 100% confidence; and if the value x is equal to 0, the blast cell may be identified as a type of cell other than a myeloid blast cell with 100% confidence. It is known that it can be challenging to differentiate between myeloid blast cells and lymphoid blast cells, and accordingly, it is conceivable that in some cases (either due to the nature of the blast cell itself or, for example, the angle at which it is shown in the digital image), it is not possible accurately to classify the cell. Accordingly, in some cases, if the numerical value falls within a predetermined range, the output of the parametric classifier is configured to be inconclusive or uncertain. Such results may be discarded. The predetermined value may be between 0.1 and 0.9, 0.2 and 0.8, 0.3 and 0.7, 0.4 and 0.6, or 0.45 and 0.55.

Each output may take the form [first value, second value]. Consider the case where the predetermined threshold range is 0.4 to 0.6:
- If the result is [0.98, 0.02], then one could conclude with 98% certainty that the blast cell in question is a lymphoid blast cell.
- If the result is [0.43, 0.57], then the result would be considered inconclusive, since the values fall within the range of 0.4 to 0.6.
- If the result is [0.22, 0.78], then one could conclude with 78% certainty that the blast cell in question is a myeloid blast cell.

In these cases, it is assumed that the blast cell is either a myeloid blast cell or a lymphoid blast cell.

Throughout the application so far, we refer to a "parametric model classifier". In the context of the present application, a parametric model is one which assumes a parametric form for a function for generating the output from the input data (i.e. the data representing the digital image), the function comprising a fixed number of parameters. The parametric form relies, as the name suggests, on a plurality of parameters, and the goal of e.g. a training process is to identify those parameters. In contrast, non-parametric models are unbounded, and there is no limit to their complexity. Advantages of parametric model classifiers (relative to non-parametric classifiers) include simplicity, speed, and the fact that they can produce reliable results on lower volumes of data. The parametric model classifier is preferably a machine learning-based classifier. The classifier is a residual neural network classifier ^{1 2 3} (herein, "ResNet"). A ResNet is an artificial neural network that builds on constructs known from pyramidal cells in the cerebral cortex. ResNets work on the principle of skip connections or shortcuts to jump over the layers in the neural network. Typical ResNet models are implemented with double- or triple-layer skips that contain nonlinearities or and batch normalization in between. There are two reasons why connections may be skipped: to avoid the problem of vanishing
¹ He, Kaiming; Zhang, Xiangyu; Ren, Shaoqing; Sun, Jian (2015-12-10). "Deep Residual Learning for Image Recognition". arXiv:1512.03385
² He, Kaiming; Zhang, Xiangyu; Ren, Shaoqing; Sun, Jian (2016). "Deep Residual Learning for Image Recognition". Proc. Computer Vision and Pattern Recognition (CVPR), IEEE.
³ Krizhevsky, Alex, Ilya Sutskever, and Geoffrey E. Hinton. "Imagenet classification with deep convolutional neural networks." Advances in neural information processing systems 25 (2012): 1097-1105. gradients, or to mitigate the degradation (accuracy saturation) problem, where adding more layers to a suitably deep model leads to higher training error. During training, the weights adapt to mute the upstream layer and amplify the previously-skipped layer. In the simplest case, only the weights for the adjacent layer's connection are adapted, with no explicit weights for the upstream layer. This works best when a single nonlinear layer is stepped over, or when the intermediate layers are all linear. If not, then an explicit weight matrix may be learned for the skipped connection. ResNets are advantageous because skipping effectively simplifies the network, using fewer layers in the initial training stages, thereby speeding learning by reducing the impact of vanishing gradients, as there are fewer layers to propagate through. The network then gradually restores the skipped layers as it learns the feature space. Towards the end of training, when all layers are expanded, it stays closer to the manifold and thus learns faster. Examples of suitable ResNets include: *ResNet34, ResNet50* and ResNet101, in which the number represents the number of layers present in the residual neural network. In variants, which are not covered by the scope of the claims, other types of convolutional neural networks such as Inception⁴, VGG⁵, and EfficientNet⁶ may also be used in implementations of the invention.
⁴ Szegedy et al. (2014) "Going Deeper with Convolutions" arXiv:1409.4842
⁵ Simonyan & Zisserman (2015) "Very Deep Convolutoinal Networks for Large-Scale Image Recognition" arXiv:1409.1556
**6** Tan & Le (2019) "EfficientNet: Rethinking Model Scaling for Convolutional Neural Networks" arXiv:1905.11946

Alternatively, or additionally, in further variants which are not covered by the scope of the claims, the model may be created using multiple instance learning, using e.g. Deep Attention MIL, as described in Ilse et al. (2018)⁷.
⁷ Maximilian Ilse, Jakub M. Tomcak, Max Welling (2018) " Attention-based Deep Multiple Instance Learning". arxiv:1802.04712

In yet another variant, which is not covered by the scope of the claims, rather than neural network methods involving training a model using per image labels, a Siamese model could be used. A Siamese model takes two images and has to determine whether they are of the same class. The labels, which apply either to a complete slide or to each cell individually, could be used to identify each pair as true if it consists of the same cell type (e.g. two lymphoid blast cells or two myeloid blast cells) or false if the images are from cells of different types.

An aim of the present invention is to improve the efficiency with which a patient may be diagnosed with either AML or ALL (or neither). We therefore now consider how the output of the parametric model classifier is used. Accordingly, the computer-implemented method may further comprise calculating a patient level score based on the respective output value x for all of the blast cells in the digital image. In this way, a diagnosis, or clinical decision may be made based on a plurality of blast cells, rather than a single one. The patient level score may comprise one or more of: a mean value, a median value, a maximum value, or a minimum value. Specifically, the patient level score may be calculated on all of the x values representing the likelihood that a blast cell is a lymphoid blast cell, and/or the likelihood that the blast cell is a myeloid blast cell. If the numerical value of the patient level score falls within a predetermined range, the output of the parametric classifier is configured to be inconclusive or uncertain. Such results may be discarded

In some cases, the computer-implemented method of the first aspect of the invention may be used as part of a decision support system to enable clinicians to select an appropriate course of action. In such cases, it may be desirable for the clinician to review the results generated by the parametric model classifier. Specifically, the computer-implemented may further comprise: generating, based on the output of the parametric model classifier, instructions configured to cause a display device of a computing system to display a gallery comprising: a first plurality of images showing the blast cells identified as lymphoid blast cells with the highest confidence; and a second plurality of images showing the blast cells identified as myeloid blast cells with the highest confidence. In this context "with the highest confidence" may be understood to mean the blast cells for which the probability of being a particular type of blast cell (i.e. a myeloid blast cell or a lymphoid blast cell) is the highest. This will enable a clinician to review the images, in order to determine a patient's prognosis, and to decide on an appropriate course of action. In some cases the first plurality of images includes the same number of images as the second plurality of images. The number may be between 1 and 100, more preferably between 5 and 50, more preferably between 10 and 25, and most preferably about 20.

As discussed earlier in this application, in order to reach a more definitive diagnosis, it is often necessary to perform a CD marker screening. Various types of CD marker screenings are available, each targeting a particular biomarker. In some cases, a clinician may identify a CD marker to use for screening based on the output of the parametric model classifier. However, in other cases, the computer-implemented method may further comprise selecting, from a plurality of available CD markers, one or more CD markers, based on the output of the parametric model classifier. In this way, a CD marker to be used for subsequent testing can be identified automatically based on the output of the parametric model.

In order for a parametric model to provide accurate results, it is often necessary to train it using known data. A second aspect of the present invention therefore provides a computer-implemented method of generating a parametric model classifier configured to differentiate between lymphoid blast cells and myeloid blast cells in a digital image, the computer-implemented method comprising: receiving a plurality of pairs of labelled training data, each pair of labelled training data including: input data comprising a digital image of a blast cell from a patient who has been diagnosed with either acute myeloid leukaemia or acute lymphoid leukaemia; and output data comprising an indication of whether the patient has acute myeloid leukaemia or acute lymphoid leukaemia; and training a parametric model classifier using the training data. The optional features set out above in respect of the first aspect of the invention apply equally well to the second aspect of the invention where compatible. For the avoidance of doubt, we stress that, in the second aspect of the invention, the parametric model classifier may be machine learning-based classifier. The classifier is residual neural network classifier (herein, "ResNet"). Examples of suitable ResNets include: *ResNet34, ResNet50* and ResNet101, in which the number represents the number of layers present in the residual neural network. The convolutional neural network may be trained using an Adam optimizer⁸. The convolutional neural network may be trained using the one-cycle policy for learning rate scheduling, as described in Smith⁹. A framework which may be used for training of the convolutional neural network is fastai¹⁰, and may use the library of Paszke et al¹¹. A different learning strategy relying instead on flat learning rates and cosine annealing could also be used. Rather than convolutional neural networks, vision transformers or experimental models such as capsules could also be used.
⁸ "Adam: A Method for Stochastic Optimization" by Kingma et al https://arxiv.org/abs/1412.6980
⁹ "A disciplined approach to neural network hyper-parameters: Part I -- learning rate, batch size, momentum, and weight decay" by Smith https://arxiv.org/abs/1803.09820
¹⁰ "fastai: A Layered API for Deep Learning" by Jeremy Howard, Sylvain Gugger https://arxiv.org/abs/2002.04688
¹¹ "PyTorch: An Imperative Style, High-Performance Deep Learning Library" by Paszke et al, https://arxiv.org/abs/1912.01703

The indication of whether the patient has acute myeloid leukaemia or acute lymphoid leukaemia may comprise a numerical value. Specifically, the numerical value may be 1 if the patient has acute myeloid leukaemia, and 0 otherwise. Conversely, the numerical value may be 1 if the patient has acute lymphoid leukaemia, and 0 otherwise. More detailed information about the training process is set out later in this application, in the "Experimental Results" section.

In the computer-implemented method of the first aspect of the invention, the parametric model classifier may be trained using the computer-implemented method of the second aspect of the invention.

A third aspect of the invention provides a computer-implemented method of generating a provisional diagnosis of acute myeloid leukaemia or acute lymphoid leukaemia, the computer-implemented method comprising: performing the computer-implemented method of the first aspect of the invention; and based on the output of the parametric model classifier or a patient level score calculated based on the output of the parametric model classifier, determining whether a patient whose blast cells are shown in the digital image is suffering from acute myeloid leukaemia, acute myeloid leukaemia, or neither. As before, all optional features set out in respect of the first aspect of the invention or the second aspect of the invention apply equally well to computer-implemented inventions of the third aspect of the invention. Furthermore, based on the result of the determination, computer-implemented methods of the third aspect of the invention may further comprise generating instructions configured to cause a display device of a computing system to display the result of the determination.

The first to third aspects of the invention relate to computer-implemented methods. Corresponding fourth to sixth aspects of the invention provide computer program products which, when the program is executed by a computer or other computing device, cause the computer to carry out the computer-implemented methods, respectively, of the first to third aspects of the invention. Seventh to ninth aspects of the invention provide, respectively, a computer-readable data carrier having stored thereon the computer program product of the fourth to sixth aspects of the invention.

As explained above, a purpose of the invention is to provide a clinical decision support system which assists clinicians in their diagnoses of acute myeloid leukaemia and/or acute lymphoid leukaemia. A tenth aspect of the invention, accordingly, provides a clinical decision support system comprising a computing device having a processor, the processor configured to perform the computer-implemented method of any one of the first to third aspects of the invention.

A particularly preferred eleventh aspect of the invention provides a computer-implemented method of differentiating between lymphoid blast cells and myeloid blast cells, the computer-implemented method comprising: receiving a digital image containing one or more blast cells; applying an image analysis to the digital image, the image analysis algorithm configured to: detect one or more blast cells in the digital image; generate a bounding box around each of the one or more blast cells; and generate a plurality of image files, each image file containing a digital image of a blast cell of the one or more blast cells, the boundary of the image in each file corresponding to a respective bounding box; applying a deep neural network classifier to each of the generated image files, the deep neural network classifier configured to generate an output indicative of whether each blast cell is a lymphoid blast cell or a myeloid blast cell, the output comprising a numerical value x in the range [0,1], wherein: **either** if the value x is equal to 1, the blast cell is identified as a lymphoid blast cell with 100% confidence, and if the value x is equal to 0, the blast cell is identified as a myeloid blast cell with 100% confidence; **or** if the value x is equal to 1, the blast cell is identified as a myeloid blast cell with 100% confidence, and if the value x is equal to 0, the blast cell is identified as a lymphoid blast cell with 100% confidence; and calculating a patient level score based on the respective output value x for all of the blast cells in the digital image, wherein the patient level score comprises a mean value; a median value; a maximum value of x; and/or a minimum value of x. Optional features set out above in respect of the first to tenth aspects of the invention apply equally well to the eleventh aspect of the invention, except where clearly technically incompatible or where context clearly dictates otherwise.

In some cases, the deep neural network classifier of the eleventh aspect of the invention is generated using a computer-implemented method comprising: receiving a plurality of pairs of labelled training data, each pair of labelled training data including: input data comprising a digital image of a blast cell from a patient who has been diagnosed with either acute myeloid leukaemia or acute lymphoid leukaemia; output data comprising an indication of whether the patient has acute myeloid leukaemia or acute lymphoid leukaemia, the output comprising a numerical value x in the taking the value 0 or 1, wherein: **either** if the value x is equal to 1, the blast cell is a lymphoid blast cell, and if the value x is equal to 0, the blast cell is a myeloid blast cell; **or** if the value x is equal to 0, the blast cell is a lymphoid blast cell, and if the value x is equal to 1, the blast cell is a myeloid blast cell; and training the deep neural network classifier using the training data.

A twelfth aspect of the invention provides a clinical decision support system, comprising: a computing device having a processor, the processor configured to generate a provisional diagnosis of acute myeloid leukaemia or acute lymphoid leukaemia by performing the computer-implemented method of the eleventh aspect of the invention and wherein the processor is further configured to: based on the patient level score calculated based on the output of the deep neural network classifier, determine whether a patient whose blast cells are shown in the digital image is suffering from acute myeloid leukaemia, acute lymphoid leukaemia, or neither; and generate, based on a result of the determination, instructions configured to cause a display device of a computing system to display the result of the determination.

### BRIEF DESCRIPTION OF THE DRAWINGS

Embodiments of the present invention will now be described with reference to the accompanying drawings, in which:
- Fig. 1 shows a system which may be used to implement computer-implemented methods according to some aspects of the invention.
- Figs. 2A and 2B shows a raw image containing blast cells which may be obtained by the imaging apparatus shown in Fig. 1.
- Fig. 3 is a flowchart illustrating a high-level method for classifying blast cells.
- Fig. 4 is an example of a view that may be produced on a display based on the output of the blast cell classification.

### DETAILED DESCRIPTION OF THE DRAWINGS

Aspects and embodiments of the present invention will now be discussed with reference to the accompanying figures. Further aspects and embodiments will be apparent to those skilled in the art.

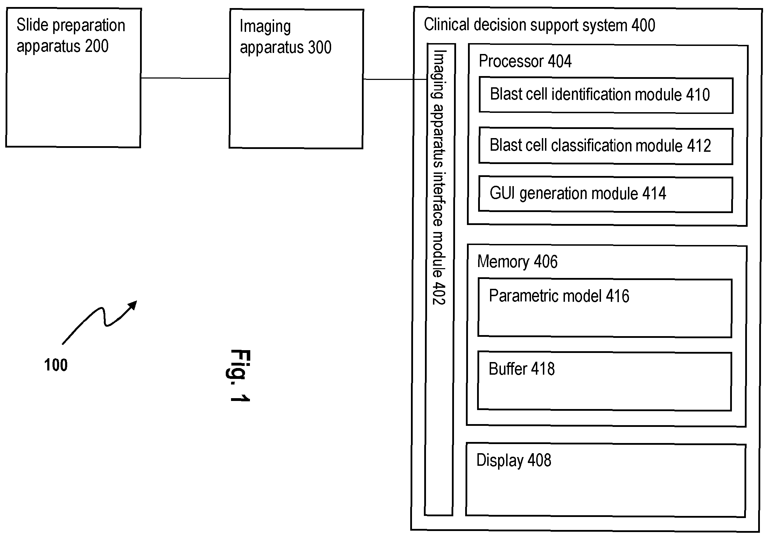

Fig. 1 shows a system 100 which includes various components for performing or enabling the performance of computer-implemented methods according to various aspects of the invention. The system 100 includes slide preparation apparatus 200, imaging apparatus 300 and a clinical decision support system 400. The slide preparation apparatus 200 is preferably configured to prepare a slide containing one or more blast cells. As discussed earlier in this application, the slide preparation may use the methods set out in WO 2012/030313 A1, US 2009/0269799 A1, US 2010/284602 A1, US 2011/014645 A1, and US 2016/209320 A1.

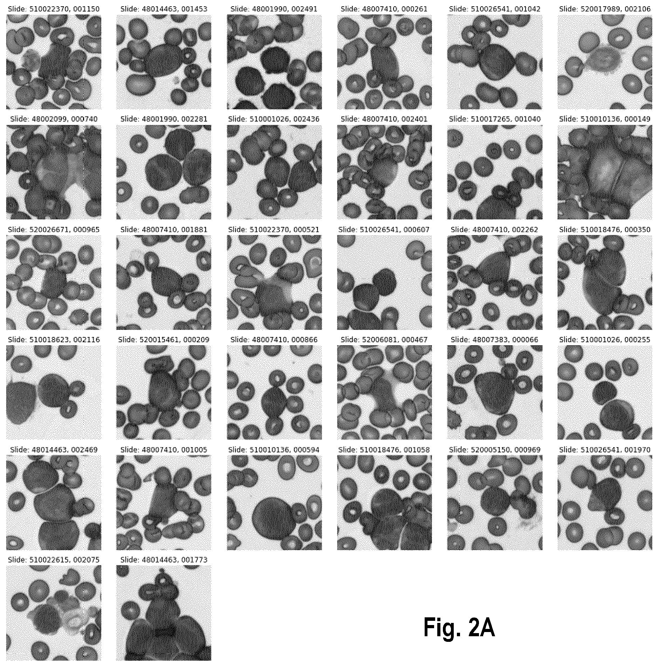

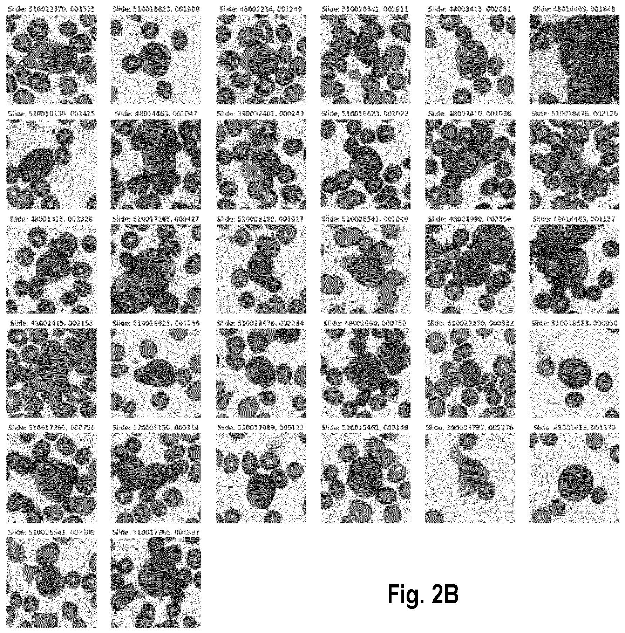

The slide preparation is outside of the scope of this application, and will not be discussed in any more detail. Once the slide has been prepared, it is imaged using the imaging apparatus 300 which may include, for example a microscope and a camera (not shown). When slide preparation apparatus 200 prepares a slide as in the references cited above, a monolayer is formed which enables each individual cell (including red blood cells, white blood cells, platelets, and crucially - blast cells) to be visualized and counted. The clinical decision support system 400 then receives the image containing blast cells from the imaging apparatus 300. Examples of the kind of images which might be received are shown in Figs. 2A and 2B. The clinical decision support system 400 is then used to identify and classify blast cells in the image. The example of the clinical decision support system shown in Fig. 1 includes an imaging apparatus interface module 402, a processor 404, a memory 406, and a display 408. It should be noted that in some alternative arrangements, the display 408 may be an external component. In those cases, the processor 404 of the clinical decision support apparatus 400 might further include a display module interface module (not shown). The processor 404 of the clinical decision support system 400 may comprise a blast cell identification module 410, a blast cell classification module 412, and graphical user interface (GUI) generation module 414. Herein, the "modules" may be in the form of physical modules, or functional modules, implemented, for example in the form of software modules (i.e. in computer-readable code). The memory 406 may comprise a parametric model 416, which may be applied to the image containing blast cells by the blast cell classification module 412. The memory may further comprise a buffer 418.

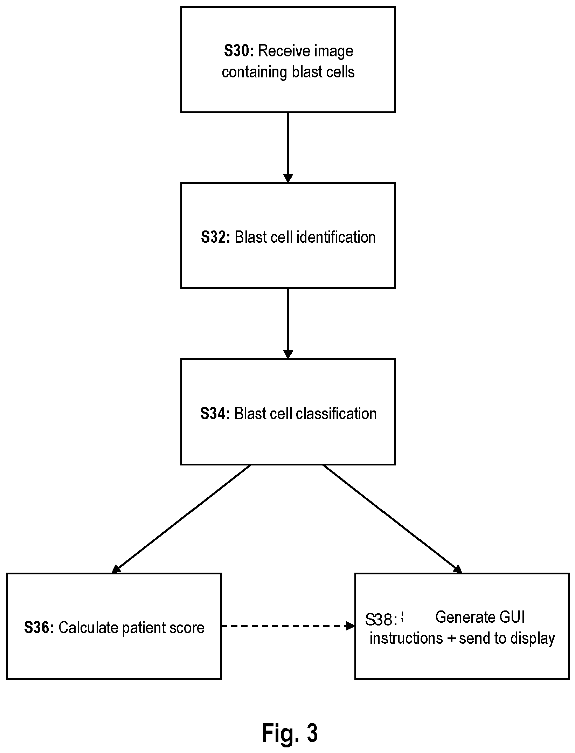

We now explain the operation of the clinical decision support apparatus 400 to identify and classify blast cells, and to generate instructions to cause various images to be displayed on the display 408. Fig. 3 is a flowchart which illustrates the high-level steps of the computer-implemented method.

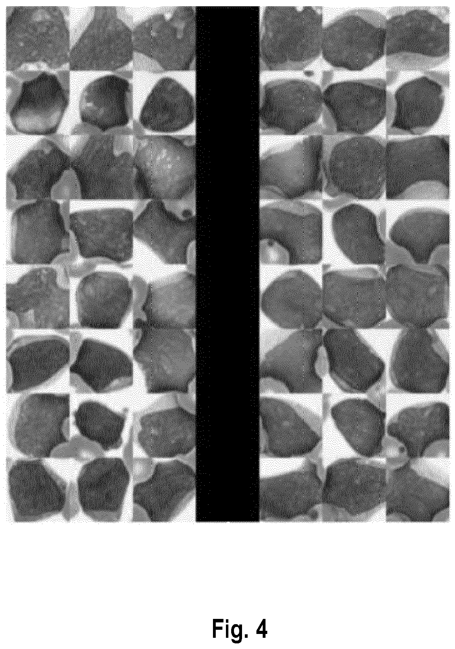

In a first step S30, the image containing the blast cells is received from the imaging apparatus 300 at the clinical decision support system 400 via the imaging apparatus interface module 402 thereof. Then, in step S32, the blast cells are identified within the image by the blast cell identification module 410. The output of this process may be, for example, several image files (which may be stored temporarily in the buffer 418 or more permanently in the memory 406), each containing an image of a single blast cell from the image. Alternatively, rather than generating a plurality of image files, the blast cell identification module 410 may identify blast cells within the image, and define boundaries of regions, each containing a single blast cell. The output of the blast cell identification module 410 may be in the form of a list of pixel arrays, each pixel array corresponding to a region of the image containing a single blast cell. This list may also be stored in the buffer 418 or more permanently in the memory 406. In step S34, the parametric model 416 is applied to each image file (or region of image containing a blast cell) by the blast cell classification module 412 in order to determine whether the blast cell is a lymphoid blast cell or a myeloid blast cell. As discussed earlier, the output of the blast cell classification module 412, for each blast cell, is preferably a number from 0 to 1, the number representing a probability or a confidence level that the blast cell in question is either a myeloid blast cell or a lymphoid blast cell. Alternatively, the model may return two probabilities, one that the blast cell is a lymphoid blast cell, and one that the blast cell is a myeloid blast cell. These probabilities should add to 1 (or 100%, or equivalent). Then, once a plurality of probabilities have been calculated using the parametric model 416, by the blast cell classification module 412, various different steps may be taken. In Fig. 3, these are represented as different branches of the flowchart, but it must be stressed that this should not be understood to mean that computer-implemented methods according to various aspects of the invention cannot include more than one of the steps on different branches. In step S36, a patient score is calculated and stored in the memory 406 (e.g. in the buffer 418). Herein, a patient level score is a statistical parameter representative of the probability values calculated for each of the images of (single) blast cells. As discussed earlier, the patient level score may take various forms. In step S38, the GUI generation module 414 may be configured to generate instructions, based on the output of the blast cell classification module 412, which when received by the display 408, cause the display to present to a user of the clinical decision support system 400 the results. For example, the display 408 may display a gallery, as shown in Fig. 4, which includes the 18 cells with the highest probability of being lymphoid blast cells, and the 20 cells with the highest probability of being myeloid blast cells. Of course, the display need not be in the form of a gallery such as this - this is just one option. In some cases, the patient level score and the individual probability for each blast cell may also be displayed.

In some examples, not shown, the method may further include a step of determining an appropriate CD marker for a subsequent screening step based on e.g. the patient level score.

### EXPERIMENTAL RESULTS

Having explained the computer-implemented method of the invention we now present some experimental data which demonstrates the efficacy of the invention. Two experiments were performed on different sets of data, referred to herein as the Toulouse Dataset and the Boston Dataset.

It will be appreciated from the below that computer-implemented methods according to various aspects of the invention are very reliable at differentiating between myeloid blast cells and lymphoid blast cells.

### A. The Toulouse Dataset

In this experiment, image from blood smears of 119 patients were used, of whom 52 had acute myeloid leukaemia, and 67 had acute lymphoid leukaemia (51 ALL-B, and 16 ALL-T). The images of the blood smears were acquired on a CellaVision instrument with high resolution.

16 slides were sampled randomly as a validation set, and the rest were used to train the neural network, in this case a *ResNet50* network.

On the validation set, an AUC (Area Under Curve in the Receiver Operator Plot) of 83.91% was achieved per cell.

After aggregating the results, i.e. by generating a patient level score, the AUC increased to 95.31%.

The same model was tested on an additional dataset from the same hospital which contained 125 slides from AML / ALL cases with lower Blast counts. On this dataset the same model achieved an AUC of 0.89963.

### B. The Boston Dataset

In this experiment, 39 slides were obtained from AML or ALL patients. The slides were printed and imaged using the methods explained earlier in this application. The images were obtained at 20× magnification using a high-resolution camera. Of the 39 slides, 21 were from AML patients, and 18 were from ALL patients. The neural network (a *ResNet50* network) was trained on 29 slides, and 10 slides (5 AML and 5 ALL) were kept as a validation set. Depending on which slides were chosen for the validation set, an AUC of 79.51% to 91.55%.

### C. Alternative training/evaluation procedure

The dataset is split into a training and validation set by excluding complete slides, rather than randomly choosing cells from the complete dataset.

To thoroughly test the per slide performance for the whole dataset a per slide cross validation was performed in which one slide was excluded, and the model was trained on all the others. The performance was the evaluated on the excluded slide. This was repeated for all the slides.

An ensemble of 5 models (again using ResNet34) was used. Ensembles deliver better performance because random mistakes from individual models are corrected. A total AUC of 86.09% was achieved, for the whole dataset.

### GENERAL STATEMENTS ABOUT THE APPLICATION

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A computer-implemented method of differentiating between lymphoid blast cells and myeloid blast cells, the computer-implemented method comprising:
receiving (S30) a digital image containing one or more blast cells;
applying an image analysis to the digital image, the image analysis algorithm configured to:
detect (S32) one or more blast cells in the digital image; and
generate a plurality of image files, each image file containing a digital image of a blast cell of the one or more blast cells; and
applying (S34) a deep neural network classifier to each of the generated image files, the deep neural network classifier being configured to generate an output indicative of whether each blast cell is a lymphoid blast cell or a myeloid blast cell, the output comprising a numerical value x in the range [0,1], wherein:
**either** if the value x is equal to 1, the blast cell is identified as a lymphoid blast cell with 100% confidence, and if the value x is equal to 0, the blast cell is identified as a myeloid blast cell with 100% confidence; **or**
if the value x is equal to 1, the blast cell is identified as a myeloid blast cell with 100% confidence, and if the value x is equal to 0, the blast cell is identified as a lymphoid blast cell with 100% confidence, **characterised in that** the deep neural network classifier is a ResNet34 classifier, a ResNet50 classifier or a ResNet101 classifier.

2. A computer-implemented method according to claim 1, wherein:
the digital image is a bright-field microscopy image containing one or more blast cells which have been Romanowsky stained.

3. A computer-implemented method according to claim 1 or claim 2, further comprising:
calculating a patient level score based on the respective output value x for all of the blast cells in the digital image, wherein the patient level score comprises:
a mean value;
a median value;
a maximum value of x; and/or
a minimum value of x.

4. A computer-implemented method according to any one of claims 1 to 3, further comprising:
generating, based on the output of the parametric model classifier, instructions configured to cause a display device of a computing system to display a gallery comprising:
a first plurality of images showing the blast cells identified as lymphoid blast cells with the highest confidence; and
a second plurality of images showing the blast cells identified as myeloid blast cells with the highest confidence.

5. A computer-implemented method according to any one of claims 1 to 4, wherein:
the image analysis algorithm is further configured to generate a bounding box around each of the one or more blast cells; and
the boundary of the image in each file corresponds to a respective bounding box.

6. A computer-implemented method according to any one of claims 1 to 5, wherein the deep neural network classifier is generated using a computer-implemented method comprising:
receiving a plurality of pairs of labelled training data, each pair of labelled training data including:
input data comprising a digital image of a blast cell from a patient who has been diagnosed with either acute myeloid leukaemia or acute lymphoid leukaemia;
output data comprising an indication of whether the patient has acute myeloid leukaemia or acute lymphoid leukaemia, the output comprising a numerical value x in the range [0,1], wherein:
**either** if the value x is equal to 1, the blast cell is identified as a lymphoid blast cell with 100% confidence, and if the value x is equal to 0, the blast cell is identified as a myeloid blast cell with 100% confidence; **or**
if the value x is equal to 1, the blast cell is identified as a myeloid blast cell with 100% confidence, and if the value x is equal to 0, the blast cell is identified as a lymphoid blast cell with 100% confidence; and
training the deep neural network classifier using the training data.

7. A clinical decision support system (400), comprising:
a computing device having a processor (404), the processor configured to generate a provisional diagnosis of acute myeloid leukaemia or acute lymphoid leukaemia by performing the computer-implemented method of any one of claims 1 to 5; and
wherein the processor is further configured to:
based on the patient level score calculated based on the output of the deep neural network classifier (S36), determine whether a patient whose blast cells are shown in the digital image is suffering from acute myeloid leukaemia, acute lymphoid leukaemia, or neither; and
generate (S38), based on a result of the determination, instructions configured to cause a display device (408) of a computing system to display the result of the determination.

## Patentansprüche

1. Computerimplementiertes Verfahren zur Unterscheidung zwischen Lymphoblasten und Myeloblasten, wobei das computerimplementierte Verfahren Folgendes umfasst:
Empfangen (S30) eines digitalen Bildes, das eine oder mehrere Blasten enthält;
Anwenden einer Bildanalyse auf das digitale Bild, wobei der Bildanalysealgorithmus zu Folgendem konfiguriert ist:
Erkennen (S32) eines oder mehrerer Blasten in dem digitalen Bild; und
Erzeugen einer Vielzahl von Bilddateien, wobei jede Bilddatei ein digitales Bild eines Blasten des einen oder der mehreren Blasten enthält; und
Anwenden (S34) eines tiefen neuronalen Netzwerk-Klassifikators auf jede der erzeugten Bilddateien, wobei der tiefe neuronale Netzwerk-Klassifikator konfiguriert ist, um eine Ausgabe zu erzeugen, die angibt, ob jeder Blast ein Lymphoblast oder ein Myeloblast ist, wobei die Ausgabe einen numerischen Wert x im Bereich [0,1] umfasst, wobei:
**entweder,** wenn der Wert x gleich 1 ist, der Blast mit 100%iger Konfidenz als ein Lymphoblast identifiziert wird, und wenn der Wert x gleich 0 ist, der Blast mit 100%iger Konfidenz als ein Myeloblast identifiziert wird; **oder,**
wenn der Wert x gleich 1 ist, der Blast mit 100%iger Konfidenz als ein Myeloblast identifiziert wird, und wenn der Wert x gleich 0 ist, der Blast mit 100%iger Konfidenz als ein Lymphoblast identifiziert wird,
**dadurch gekennzeichnet, dass** der tiefe neuronale Netzwerk-Klassifikator ein ResNet34-Klassifikator, ein ResNet50-Klassifikator oder ein ResNet101-Klassifikator ist.

2. Computerimplementiertes Verfahren nach Anspruch 1, wobei:
das digitale Bild ein Hellfeldmikroskopiebild ist, das eine oder mehrere Blasten enthält, die mit Romanowsky-Färbung gefärbt wurden.

3. Computerimplementiertes Verfahren nach Anspruch 1 oder Anspruch 2, ferner umfassend:
Berechnen eines patientenbezogenen Scores, basierend auf dem jeweiligen Ausgabewert x, für alle der Blasten in dem digitalen Bild, wobei der patientenbezogene Score Folgendes umfasst:
einen Mittelwert;
einen Medianwert;
einen Maximalwert von x; und/oder
einen Minimalwert von x.

4. Computerimplementiertes Verfahren nach einem der Ansprüche 1 bis 3, ferner umfassend:
Erzeugen von Anweisungen, die konfiguriert sind, um eine Anzeigevorrichtung eines Computersystems zu veranlassen, eine Galerie anzuzeigen, basierend auf der Ausgabe des Klassifikators für parametrische Modelle, umfassend:
eine erste Vielzahl von Bildern, die die Blasten zeigen, die mit der höchsten Konfidenz als Lymphoblasten identifiziert wurden; und
eine zweite Vielzahl von Bildern, die die Blasten zeigen, die mit der höchsten Konfidenz als Myeloblasten identifiziert wurden.

5. Computerimplementiertes Verfahren nach einem der Ansprüche 1 bis 4, wobei:
der Bildanalysealgorithmus ferner konfiguriert ist, um einen Begrenzungsrahmen um jeden des einen oder der mehreren Blasten zu erzeugen; und
die Grenze des Bildes in jeder Datei einem jeweiligen Begrenzungsrahmen entspricht.

6. Computerimplementiertes Verfahren nach einem der Ansprüche 1 bis 5, wobei der tiefe neuronale Netzwerk-Klassifikator unter Verwendung eines computerimplementierten Verfahrens erzeugt wird, umfassend:
Empfangen einer Vielzahl von Paaren von markierten Trainingsdaten, wobei jedes Paar von markierten Trainingsdaten Folgendes einschließt:
Eingabedaten, die ein digitales Bild eines Blasten von einem Patienten umfassen, bei dem entweder akute myeloische Leukämie oder akute lymphoide Leukämie diagnostiziert wurde;
Ausgabedaten, die eine Angabe umfassen, ob der Patient akute myeloische Leukämie oder akute lymphoide Leukämie hat, wobei die Ausgabe einen numerischen Wert x im Bereich [0,1] umfasst, wobei:
**entweder,** wenn der Wert x gleich 1 ist, der Blast mit 100%iger Konfidenz als ein Lymphoblast identifiziert wird, und wenn der Wert x gleich 0 ist, der Blast mit 100%iger Konfidenz als ein Myeloblast identifiziert wird; **oder,**
wenn der Wert x gleich 1 ist, der Blast mit 100%iger Konfidenz als ein Myeloblast identifiziert wird, und wenn der Wert x gleich 0 ist, der Blast mit 100%iger Konfidenz als ein Lymphoblast identifiziert wird; und
Trainieren des tiefen neuronalen Netzwerk-Klassifikators unter Verwendung der Trainingsdaten.

7. Klinisches Entscheidungsunterstützungssystem (400), umfassend:
eine Computervorrichtung mit einem Prozessor (404), wobei der Prozessor konfiguriert ist, um eine vorläufige Diagnose von akuter myeloischer Leukämie oder akuter lymphoider Leukämie durch Durchführen des computerimplementierten Verfahrens nach einem der Ansprüche 1 bis 5 zu erzeugen; und
wobei der Prozessor ferner für Folgendes konfiguriert ist:
Bestimmen, ob ein Patient, dessen Blasten in dem digitalen Bild gezeigt werden, an akuter myeloischer Leukämie, akuter lymphoider Leukämie oder keiner von beiden leidet, basierend auf dem patientenbezogenen Score, der basierend auf der Ausgabe des tiefen neuronalen Netzwerk-Klassifikators (S36) berechnet wurde; und
Erzeugen (S38), basierend auf einem Ergebnis der Bestimmung, von Anweisungen, die konfiguriert sind, um eine Anzeigevorrichtung (408) eines Computersystems zu veranlassen, das Ergebnis der Bestimmung anzuzeigen.

## Revendications

1. Procédé mis en œuvre par ordinateur de différenciation entre les cellules blastiques lymphoïdes et les cellules blastiques myéloïdes, le procédé mis en œuvre par ordinateur comprenant :
la réception (S30) d'une image numérique contenant une ou plusieurs cellules blastiques ;
l'application d'une analyse d'image à l'image numérique, l'algorithme d'analyse d'image étant configuré pour :
détecter (S32) une ou plusieurs cellules blastiques dans l'image numérique ; et
générer une pluralité de fichiers d'image, chaque fichier d'image contenant une image numérique d'une cellule blastique parmi la ou les cellules blastiques ; et
l'application (S34) d'un classifieur à réseau neuronal profond à chacun des fichiers d'image générés, le classifieur à réseau neuronal profond étant configuré pour générer une sortie indiquant si chaque cellule blastique est une cellule blastique lymphoïde ou une cellule blastique myéloïde, la sortie comprenant une valeur numérique x dans la plage [0,1], dans lequel :
**soit** si la valeur x est égale à 1, la cellule blastique est identifiée comme étant une cellule blastique lymphoïde avec une confiance de 100 %, et si la valeur x est égale à 0, la cellule blastique est identifiée comme étant une cellule blastique myéloïde avec une confiance de 100 % ; **soit**
si la valeur x est égale à 1, la cellule blastique est identifiée comme étant une cellule blastique myéloïde avec une confiance de 100 %, et si la valeur x est égale à 0, la cellule blastique est identifiée comme étant une cellule blastique lymphoïde avec une confiance de 100 %,
**caractérisé en ce que** le classifieur à réseau neuronal profond est un classifieur ResNet34, un classifieur ResNet50 ou un classifieur ResNet101.

2. Procédé mis en œuvre par ordinateur selon la revendication 1, dans lequel :
l'image numérique est une image par microscopie en fond clair contenant une ou plusieurs cellules blastiques qui ont été colorées selon Romanowsky.

3. Procédé mis en œuvre par ordinateur selon la revendication 1 ou 2, comprenant en outre :
le calcul d'un score de niveau du patient en se basant sur la valeur de sortie x respective pour toutes les cellules blastiques dans l'image numérique, dans lequel le score de niveau du patient comprend :
une valeur moyenne ;
une valeur médiane ;
une valeur maximale de x ; et/ou
une valeur minimale de x.

4. Procédé mis en œuvre par ordinateur selon l'une quelconque des revendications 1 à 3, comprenant en outre :
la génération, en se basant sur la sortie du classifieur à modèle paramétrique, d'instructions configurées pour amener un dispositif d'affichage d'un système informatique à afficher une galerie comprenant :
une première pluralité d'images montrant les cellules blastiques identifiées comme étant des cellules blastiques lymphoïdes avec la confiance la plus élevée ; et
une seconde pluralité d'images montrant les cellules blastiques identifiées comme étant des cellules blastiques myéloïdes avec la confiance la plus élevée.

5. Procédé mis en œuvre par ordinateur selon l'une quelconque des revendications 1 à 4, dans lequel :
l'algorithme d'analyse d'image est en outre configuré pour générer une boîte englobante autour de chacune de la ou des cellules blastiques ; et
la limite de l'image dans chaque fichier correspond à une boîte englobante respective.

6. Procédé mis en œuvre par ordinateur selon l'une quelconque des revendications 1 à 5, dans lequel le classifieur à réseau neuronal profond est généré en utilisant un procédé mis en œuvre par ordinateur comprenant :
la réception d'une pluralité de paires de données d'entraînement marquées, chaque paire de données d'entraînement marquées comprenant :
des données d'entrée comprenant une image numérique d'une cellule blastique provenant d'un patient qui a été diagnostiqué avec soit une leucémie myéloïde aiguë soit une leucémie lymphoïde aiguë ;
des données de sortie comprenant une indication de si le patient présente une leucémie myéloïde aiguë ou une leucémie lymphoïde aiguë, la sortie comprenant une valeur numérique x dans la plage [0,1], dans lequel :
**soit** si la valeur x est égale à 1, la cellule blastique est identifiée comme étant une cellule blastique lymphoïde avec une confiance de 100 %, et si la valeur x est égale à 0, la cellule blastique est identifiée comme étant une cellule blastique myéloïde avec une confiance de 100 % ; **soit**
si la valeur x est égale à 1, la cellule blastique est identifiée comme étant une cellule blastique myéloïde avec une confiance de 100 %, et si la valeur x est égale à 0, la cellule blastique est identifiée comme étant une cellule blastique lymphoïde avec une confiance de 100 % ; et
l'entraînement du classifieur à réseau neuronal profond en utilisant les données d'entraînement.

7. Système d'aide à la décision clinique (400), comprenant :
un dispositif de calcul ayant un processeur (404), le processeur étant configuré pour générer un diagnostic provisoire de leucémie myéloïde aiguë ou de leucémie lymphoïde aiguë en réalisant le procédé mis en œuvre par ordinateur selon l'une quelconque des revendications 1 à 5 ; et
dans lequel le processeur est en outre configuré pour :
en se basant sur le score de niveau du patient calculé en se basant sur la sortie du classifieur à réseau neuronal profond (S36), déterminer si un patient dont les cellules blastiques sont montrées dans l'image numérique souffre de leucémie myéloïde aiguë, de leucémie lymphoïde aiguë, ou d'aucune des deux ; et
générer (S38), en se basant sur un résultat de la détermination, des instructions configurées pour amener un dispositif d'affichage (408) d'un système informatique à afficher le résultat de la détermination.
